# EUROPEAN PATENT SPECIFICATION

(11) **EP 1 712 251 B1**
(45) Date of publication and mention of the grant of the patent: **05.05.2010**
(21) Application number: 06112479.8
(22) Date of filing: 11.04.2006
(51) Int. Cl.: A61M 25/09

(54) **Guide Wire**
Führungsdraht
Fil de guidage

(30) Priority: 15.04.2005 JP 2005118613; 23.05.2005 JP 2005150263
(43) Date of publication of application: 18.10.2006
(73) Proprietor: TERUMO KABUSHIKI KAISHA, Tokyo (JP)
(72) Inventor: Tano, Yutaka, Fujinomiya-shi Shizuoka (JP); Umeno, Akihiko, Fujinomiya-shi Shizuoka (JP)
(74) Representative: TBK-Patent

(56) References cited:
- EP-A- 1 388 348
- EP-A- 1 388 349
- EP-A- 1 388 350
- EP-A- 1 391 216
- EP-A- 1 543 857
- US-A1- 2004 106 878

## Description

### BACKGROUND OF THE INVENTION

The present invention relates to a guide wire, particularly to a guide wire used in introducing a catheter into a body lumen such as a blood vessel and a bile duct.

Guide wires are used to guide a catheter in treatment of cites at which open surgeries are difficult or which require low invasiveness to the body, for example, PTCA (Percutaneous Transluminal Coronary Angioplasty), or in examination such as cardioangiography. A guide wire used in the PTCA procedure is inserted, with the distal end projecting from the distal end of a balloon catheter, into the vicinity of a target angiostenosis portion together with the balloon catheter, and is operated to guide the distal end portion of the balloon catheter to the target angiostenosis portion.

Furthermore, a guide wire is used, in treatment of a lesion portion of the bile duct or pancreatic duct, to guide each of various treatment devices to the vicinity of the lesion portion of the bile duct or pancreatic duct by, for example, the following methods.

### [1] ERCP (endoscopic retrograde cholangiopancreatography)

A method in which an endoscope is inserted into the descending part of duodenum. While endoscopically observing the Vater papilla in front, a cannula is inserted into the bile duct and pancreatic duct, a contrast medium is fed in, and radiography is performed.

### [2] EST (endoscopic sphincterotomy)

A method in which a papillotome is inserted into the papilla orifice of duodenum, and incision of the papillary sphincter is performed by use of highfrequency wave.

### [3] EPBD (endoscopic papillary balloon dilation)

A method in which a papilla is dilated by a balloon through an endoscope, and a biliary calculus is removed.

A blood vessel requiring PTCA is complicatedly curved. Therefore, a guide wire used to insert a balloon catheter into such a blood vessel requires appropriate flexibility and restoring performance against bending, pushability and torque response (generically called "steerability") for transmitting an operational force from the proximal end portion to the distal side, kink resistance (resistance against sharp bending), and the like. To obtain appropriate flexibility as one of the above-described performances, there has been known a guide wire configured so that a metal coil having flexibility is provided around a small-sized core member at the distal end of the guide wire, or a guide wire including a core member made from a superelastic material such as an Ni-Ti alloy for imparting the flexibility and restoring performance.

Conventionally, many of the guide wires have a configuration in which a single core member extends from the proximal end to the distal end thereof, and a material having a comparatively high elastic modulus is used for the core member so as to enhance the steerability. This configuration, however, has tended to loose the flexibility at a distal end portion of the guide wire. On the other hand, when a material having a comparatively low elastic modulus is used for the core member in order to obtain flexibility at a distal end portion of the guide wire, the steerability on the proximal side of the guide wire is lost. Thus, it has been considered to be difficult to simultaneously obtain both the flexibility and the steerability required.

In addition, the guide wire used in the procedure by way of an endoscope is used under bad conditions such as the curving of the endoscope in a body lumen, the bending of the guide wire on the forceps riser base, and the exchange with a catheter in the condition where a contrast medium is firmly adhered, so that the torque response and the pushability of the guide wire would be spoiled. Besides, depending on the devices to be combined with the guide wire, it may be necessary to use a guide wire having a small outside diameter. As the outside diameter becomes smaller, however, the rigidity of the guide wire is lowered, and the torque response and the pushability are lowered, making it difficult to guide each treatment device to a target site.

In order to overcome these drawbacks, there has been proposed a guide wire in which, for example, an Ni-Ti alloy filament is used as the core member, and the distal side and the proximal side of the core member are heat treated under different conditions so as to enhance the flexibility of a distal end portion and to enhance the rigidity on the proximal side (see, for example, Japanese Patent Publication No. Hei 4-60675).

However, such a control of flexibility by heat treatment has a limitation. In some cases, it has been impossible to obtain satisfactory rigidity on the proximal side even though sufficient flexibility is obtained at a distal end portion.

In addition, there has been proposed a guide wire which includes a flexible first wire disposed on the distal side, a high-rigidity second wire disposed on the proximal side, and a tubular connection member connecting the first and second wires to each other and having a groove and a slit, wherein the connection member is gradually increased in rigidity from the distal side toward the proximal side (see, for example, Japanese Patent Laid-open No. Hei 10-118005).

Such a guide wire can dispose wires having desired characteristics on its distal side and proximal side. However, it is difficult to provide a high joint strength of the wires, because the both wires are connected each other through a tubular connection member. Therefore, it is difficult to obtain the torque response sufficiently. Also, there is a problem in manufacturing that connection operation of the wires takes extra efforts.

EP 1 388 348 A1, EP 1 388 349 A1, EP 1 388 350 A1, EP 1 391 216 A1, EP 1 543 857 A1 and US 2004/0106878 A1 disclose further examples of conventional guide wires.
Among these guide wires, the guide wire shown in Fig. 5 of EP 1 391 216 A1 has the features defined in the preamble of claim 1. In the procedure of joining the first wire and the second wire, a fused layer is formed between the first wire and the second wire by butt welding. At his time, a projection of molten and then solidified matter is formed on the fused portion, which projects in the outer peripheral direction.

### SUMMARY OF THE INVENTION

There is a need for the present invention to provide a guide wire which has excellent pushability, torque response, and kink resistance.

In order to attain the above need, there is provided a guide wire as defined in claim 1.

The dependent claims define further developments of the invention.

According to the guide wire of the present invention, the guide wire can show excellent steerability. Furthermore the first wire rich in flexibility and the second wire higher in rigidity than the first wire are joined to each other by welding, whereby flexibility can be sufficiently secured on the distal side of the guide wire to thereby enhance safety, sufficient rigidity can be obtained on the proximal side of the guide wire, and a guide wire excellent in pushability, torque response and trackability can be obtained. These effects can be displayed not only in the cases of guide wires having ordinary outside diameters but also in the cases of guide wires having small outside diameters. Accordingly, the guide wire of the present invention shows excellent steerability in, for example, a curved catheter, an endoscope, and a body lumen such as a blood vessel, the bile duct, and the pancreatic duct.

Since the difference in rigidity between the first wire and the second wire is due mainly to the difference in outside diameter between the wires, both the wires can be formed of the same material or formed of materials of the same type. As a result, the joint strength between the first wire and the second wire can be enhanced. Therefore, even where a bending, tensile or other stress is exerted on the guide wire, the joined portion between the first wire and the second wire would not come off, so that the guide wire is high in reliability.

Particularly, where the first wire and the second wire are joined (connected) to each other by welding and the metallic coating layer is formed around the welded portion, the joint strength at the welded portion is more enhanced, making it possible to transmit more securely a torsion or the pushing-in force from the second wire to the first wire.

The presence of the metallic coating layer around the welded portion ensures that, when a bending or torsion is exerted on the guide wire, the stress is dispersed into the periphery of the welded portion, so that stress concentration on the welded portion is prevented. Therefore, the flexural or torsional stress is smoothly transmitted across the welded portion, so that it is possible to effectively prevent sharp kinking (sharp bending), torsion or the like from being generated.

Where the metallic coating layer has been reshaped by mechanically processing a solidified matter of a molten metal generated upon the welding of the first wire and the second wire, the manufacturing of the guide wire is easier and the adhesion of the metallic coating layer to the first wire and the second wire is stronger, as compared with the case where the metallic coating layer is composed of separate members, so that breakage or the like would not occur even when an excessive stress is exerted thereon. Particularly, since the metallic coating layer is composed of the material(s) of the first wire and/or the second wire, the metallic coating layer is securely adhered to the first wire and the second wire, the reshaping work can be easily carried out, and the wire body can be provided with a stepless continuous surface across the welded portion.

An example of the guide wire provides a second wire that is higher in rigidity than the first wire and joined to the first wire, whereby it is ensured that even a guide wire small in outside diameter is more flexible on the distal side, while maintaining a high rigidity on the proximal side. Therefore, a sufficient pushing-in force and a sufficient torque response can be obtained, and the guide wire shows excellent steerability in a curved catheter, in an endoscope, and in a body lumen such as a blood vessel, the bile duct and the pancreatic duct.

Where a resin coating layer, particularly, a resin coating layer formed of a material capable of reducing friction is provided, the sliding performance of the guide wire in a catheter or the like is enhanced, and the steerability of the guide wire is further enhanced. With the sliding resistance of the guide wire reduced, it is possible to prevent more securely a kinking or torsion of the guide wire, particularly, a kinking or torsion in the vicinity of the welded portion.

In the case where a resin coating layer formed of a material rich in flexibility is provided at the outer periphery of the first wire, particularly where the distal end of the first wire is covered with the resin coating layer instead of being exposed, damaging of a blood vessel inside wall or the like can be prevented more assuredly from occurring at the time of insertion of the guide wire into a blood vessel or the like, so that safety can be further enhanced.

The above and other objects, features and advantages of the present invention will become apparent from the following description and appended claims, taken in conjunction with the accompanying drawings.

### BRIEF DESCRIPTION OF THE DRAWINGS

Fig. 1 is a vertical sectional view showing an embodiment of the guide wire according to the present invention;
Fig. 2 is a vertical sectional view showing in an enlarged form the vicinity of a joined portion of a wire body in the guide wire according to the present invention;
Fig. 3 is a vertical sectional view showing another embodiment of the guide wire according to the present invention;
Fig. 4 is a schematic diagram for illustrating an example of use of the guide wire according to the present invention; and
Fig. 5 is a schematic diagram for illustrating an example of use of the guide wire according to the present invention.

### DETAILED DESCRIPTION OF THE PREFERRED EMBODIMENTS

The guide wire according to the present invention will be described in detail below, based on preferred embodiments shown in the accompanying drawings.

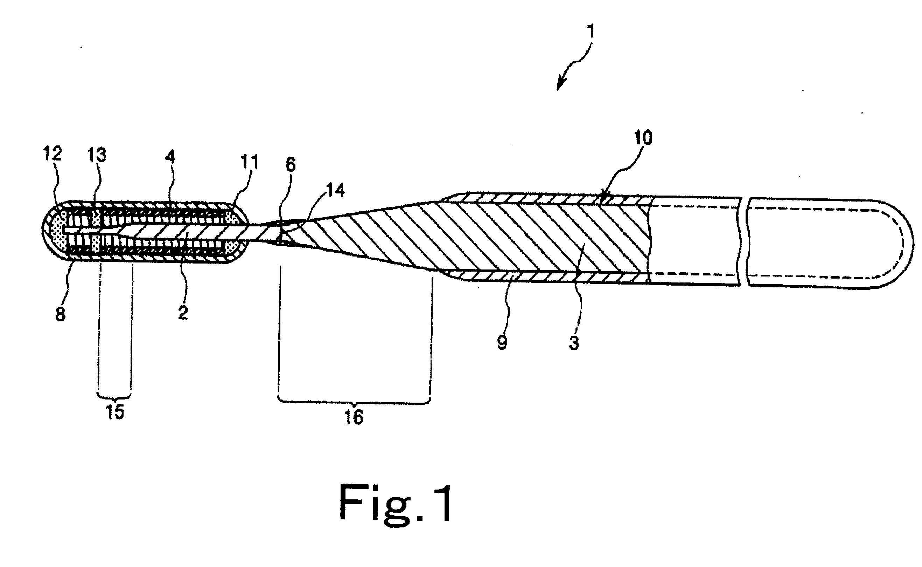

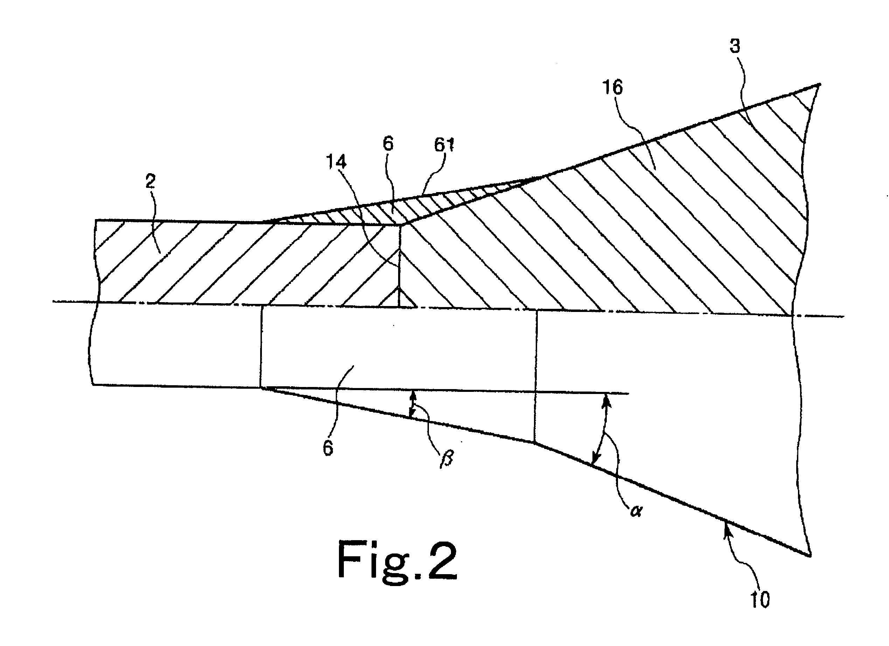

Fig. 1 is a vertical sectional view showing an embodiment of the guide wire according to the present invention, and Fig. 2 is a vertical sectional view showing in an enlarged form the vicinity of a joined portion of a wire body in the guide wire according to the present invention. Incidentally, for convenience of description, the right side in Figs. 1 and 2 is referred to as "the proximal end (proximal side)", and the left side as "the distal end (distal side)". Besides, in Figs. 1 and 2, for easy understanding, the guide wire is schematically shown by shortening the guide wire in the longitudinal direction and exaggerating the guide wire in the transverse direction. Therefore, the ratio of size in the longitudinal direction to the size in the transverse direction is different from the real ratio.

The guide wire 1 shown in Fig. 1 is a catheter guide wire used in the state of being inserted in the lumen of a catheter (inclusive of endoscope), and includes a wire body 10 and a spiral coil 4. The wire body 10 includes a first wire 2 disposed on the distal side, and a second wire 3 disposed on the proximal side of the first wire 2, the wires joined (connected) to each other by welding. The overall length of the guide wire 1 is not particularly limited, and is preferably about 200 to 5000 mm.

The first wire 2 is composed of a filamentous material having flexibility or elasticity. The length of the first wire 2 is not particularly limited, and is preferably about 20 to 1000 mm.

In this embodiment, the first wire 2 has a portion having a constant outside diameter, and a tapered portion (gradually decreasing outside diameter portion) the outside diameter of which is gradually decreased in the distal direction. The latter portion may be present in one position or in two or more positions; in the embodiment shown in the drawings, a gradually decreasing outside diameter portion 15 is provided at one position.

With the gradually decreasing outside diameter portion 15 provided, the rigidity (flexural rigidity, torsional rigidity) of the first wire 2 can be gradually reduced in the distal direction. As a result, the guide wire 1 can have good flexibility at its distal end portion, whereby trackability and safety in relation to a blood vessel or the like are enhanced, and kinking (sharp bending) or the like can be prevented from occurring.

The taper angle (outside diameter reduction rate) of the gradually decreasing outside diameter portion 15 may be constant along the longitudinal direction of the guide wire 1, or may vary along the longitudinal direction at some portions. For example, portions with a comparatively larger taper angle (outside diameter reduction rate) and portions with a comparatively smaller taper angle may be alternately repeated a plurality of times.

The portion on the proximal side of the first wire 2 (the portion on the proximal side relative to the gradually decreasing outside diameter portion 15) has an outside diameter which is constant up to the proximal end of the first wire 2.

To the proximal end of the first wire 2, the distal end of the second wire 3 is joined (connected) by, for example, welding. The distal end of the second wire 3 may be joined by soldering or brazing. The second wire 3 is composed of a filamentous material having flexibility or elasticity. The length of the second wire 3 is not particularly limited, and is preferably about 20 to 4800 mm.

A distal end portion of the second wire 3 is provided with a tapered portion 16 the outside diameter of which is gradually increased in the proximal direction from the distal end thereof (the distalmost end of the second wire 3). On the proximal side relative to the tapered portion 16 of the second wire 3, the outside diameter of the second wire 3 is substantially constant along the longitudinal direction of wire. The outside diameter of the distal end of the second wire 3 (the distal end of the tapered portion 16) is equal to the outside diameter of the proximal end of the first wire 2.

The outside diameter of the distal end of the second wire 3 (the distal end of the tapered portion 16) may be larger than the outside diameter of the proximal end of the first wire 2. The outside diameter of the distal end of the second wire 3 (the distal end of the tapered portion 16) may be smaller than the outside diameter of the proximal end of the first wire 2.

With the average outside diameter of the first wire 2 thus set smaller than the average outside diameter of the second wire 3, the guide wire 1 is rich in flexibility at its first wire 2 on the distal side and comparatively high in rigidity at its second wire 3 on the proximal side, so that the flexibility at a distal end portion and the excellent steerability (pushability, torque response) can both be attained simultaneously. In addition, with the tapered portion 16 provided at a distal end portion of the second wire 3, physical characteristics (particularly, elasticity) vary smoothly from the second wire 3 to the first wire 2, so that excellent pushability and torque response are displayed across a joined portion (joint surface) 14 between both wires 2 and 3, and kink resistance is also enhanced.

The materials constituting the first wire and the second wire are not particularly limited; as the constituent materials, there can be used various metallic materials including, for example, stainless steels (e.g., all SUS steels such as SUS304, SUS303, SUS316, SUS316L, SUS3I6J1, SUS316J1L, SUS405, SUS430, SUS434, SUS444, SUS429, SUS430F, SUS302, etc.), piano wire, Ni-Ti base alloys, and cobalt alloys. Among these materials, particularly preferred are Ni-Ti base alloys, and more preferred are Ni-Ti base pseudo-elastic alloys (inclusive of Ni-Ti base superelastic alloys).

Superelastic alloys are comparatively flexible, have restoring property, and are less susceptible to unremovable bend. When the first wire 2 is formed of a superelastic alloy, the guide wire can have sufficient flexibility and restoring property against bend in its distal side portion, and is enhanced in the property of following up to a complicatedly curved or bent blood vessel, so that more excellent steerability can be obtained. In addition, even if the first wire 2 is deformed (curved or bent) repeatedly, unremovable bends are obviated by the restoring property of the first wire 2, so that a lowering in steerability can be prevented from occurring due to an unremovable bend imparted to the first wire 2 in use of the guide wire 1.

The pseudo-elastic alloys include all shapes of tensile stress-strain curves, include those of which the transformation points such as As, Af, Ms, and Mf can be measured conspicuously as well as those of which the transformation points cannot be measured, and include all of those which are largely deformed (strained) under a stress and which can substantially return to the original shape thereof upon removal of the stress.

Examples of the preferable composition of the superelastic alloys include Ni-Ti alloys such as Ni-Ti alloys containing 49-52 at % of Ni, Cu-Zn alloys containing 38.5-41.5 wt% of Zn, Cu-Zn-X alloys (X is at least one selected from the group including Be, Si, Sn, Al, and Ga) containing 1-10 wt% of X, and Ni-Al alloys containing 36-38 at % of Al. Among these alloys, particularly preferred are the Ni-Ti based alloys. Incidentally, the superelastic alloys represented by Ni-Ti based alloys are excellent also in adhesion to resin coating layers 8 and 9 which will be described later.

Cobalt alloys are high in elastic modulus when formed into wire, and have appropriate elastic limits. Therefore, a wire formed of a cobalt alloy is excellent in torque response, and is extremely highly resistant against buckling or the like. As the cobalt alloy, any alloys can be used that contain Co as a constituent element. Among these alloys, preferred are those containing Co as a principal constituent (Co-based alloys: alloys in which the Co content by weight is the highest of the contents of constituent elements), and more preferred are Co-Ni-Cr alloys. When an alloy with such a composition is used, the above-mentioned effects are displayed more conspicuously. In addition, an alloy with such a composition has a high elastic modulus, and can be cold formed when conditioned to have a high elastic limit, so that the alloy with its high elastic limit can be formed to have a reduced diameter while sufficiently preventing the generation of buckling, and can be provided with sufficient flexibility and rigidity for insertion thereof to a predetermined site.

As the Co-Ni-Cr alloys, preferred examples include alloys containing 28-50 wt% of Co, 10-30 wt% of Ni, 10-30 wt% of Cr, and the remainder of Fe, and alloys obtained by replacing a part of these alloys by other elements (substituent elements). When the alloys contain the substituent elements, the alloys display intrinsic effects according to the kinds of the substituent elements. For example, at least one selected from the group including Ti, Nb, Ta, Be, and Mo is contained as the substituent element, a further enhancement of the strength of the second wire 3 or the like can be contrived. Incidentally, when other elements than Co, Ni, and Cr are contained, the total content of the other elements (substituent elements) is preferably not more than 30 wt%.

In addition, a part of Co, Ni, or Cr may be replaced by other element(s). For example, a part of Ni may be replaced by Mn. This makes it possible, for example, to contrive a further improvement of workability or the like. Besides, a part of Cr may be replaced by Mo and/or W. This makes it possible to contrive a further improvement of elastic limit or the like. Among the Co-Ni-Cr alloys, particularly preferred are those containing Mo, namely, Co-Ni-Cr-Mo alloys.

Specific examples of the composition of Co-Ni-Cr alloy include [1] 40 wt%Co-22wt%Ni-25wt%Cr-2wt%Mn-0.17wt%C-0.03wt%Be-remainder Fe, [2] 40wt%Co-15wt%Ni-20wt%Cr-2wt%Mn-7wt%Mo-0.15wt%C-0.03wt%Be-remainder Fe, [3] 42wt%Co-13wt%Ni-20wt%Cr-1.6wt%Mn-2wt%Mo-2.8wt%W-0.2wt%C-0.04wt%Be-remainder Fe, [4] 45wt%Co-21wt%Ni-18wt%Cr-1wt%Mn-4wt%Mo-1wt%Ti-0.02wt%C-0.3wt%Be-remainder Fe, and [5] 34wt%Co-21wt%Ni-14wt%Cr-0.5wt%Mn-6wt%Mo-2.5wt%Nb-0.5wt%Ta-remainder Fe. The Co-Ni-Cr alloys in the present invention mean the concept including these alloys.

The first wire 2 and the second wire 3 may be composed of different materials, but they may be composed of the same metallic material or of metallic materials of the same type (alloys in which the main metallic materials are the same). This ensures that the joint strength at the joined portion (welded portion) 14 is further higher, separation would not occur even when the outside diameter of the joined portion 14 is small, and excellent torque response and the like are displayed.

In this case, the first wire 2 and the second wire 3 are each preferably formed of an Ni-Ti alloy. In addition, the first wire 2 and the second wire 3 are each preferably formed of a superelastic alloy. This makes it possible to secure excellent flexibility on the distal side relative to the tapered portion 16 of the wire body 10, and to secure sufficient rigidity (flexural rigidity, torsional rigidity) at a proximal side portion of the wire body 10. As a result, the guide wire 1 has excellent pushability and torque response, thereby securing good steerability, and at the same time, has good flexibility and restoring property on the distal side, whereby trackability and safety in relation to a blood vessel, the bile duct, and the pancreatic duct are enhanced.

In the case where the first wire 2 and the second wire 3 are formed of different materials, it is preferable that the first wire 2 and the second wire 3 contain a common element. For example, the first wire 2 is formed of an Ni-Ti alloy, and the second wire 3 is formed of a stainless steel, the common element being nickel. When both wires thus contain the common element, strength of welding therebetween is enhanced. In another embodiment, the first wire 2 is formed of an Ni-Ti alloy, and the second wire 3 is formed of a Co-Ni-Cr alloy, the common element being nickel. Preferably, the first wire 2 is formed of a material which is lower in elastic modulus than the material constituting the second wire 3.

A coil 4 is disposed around a distal end portion of the first wire 2. The coil 4 is a member obtained by winding a filamentous material (thin wire) spirally, and is so disposed as to cover at least a distal side portion of the first wire 2. In the configuration shown in the drawings, the distal side portion of the first wire 2 is inserted in a substantially central portion inside the coil 4. In addition, the distal side portion of the first wire 2 is inserted so as not to make contact with the inside surface of the coil 4. The joined portion 14 is located on the proximal side relative to the proximal end of the coil 4.

Incidentally, in the configuration shown in the drawings, the coil 4 has a configuration in which, in the absence of external force, a slight gap is left between the adjacent loops of the filamentous material wound spirally. Different from this configuration, however, a configuration may be adopted in which, in the absence of external force, the loops of the filamentous material wound spirally may be densely disposed with no gap therebetween.

The coil 4 is preferably formed of a metallic material. Examples of the metallic material constituting the coil 4 include stainless steels, superelastic alloys, cobalt alloys, noble metals such as gold, platinum, tungsten, etc. and alloys containing noble metals (e.g., platinum-iridium alloy). Particularly, where the coil 4 is formed of a radiopaque material such as a noble metal, the guide wire 1 can have a radiographically effective property, providing the merit that the guide wire 1 can be inserted into a living body while radioscopically confirming the position of a distal end portion thereof. In addition, the coil 4 may be formed of different materials on the distal side and on the proximal side thereof. For example, the distal side of the coil 4 may be composed of a coil of a radiopaque material, while the proximal side of the coil 4 may be composed of a coil of a material (stainless steel or the like) comparatively transmitting X rays therethrough. Incidentally, the overall length of the coil 4 is not particularly limited, and is preferably about 5 to 500 mm.

A proximal end portion and a distal end portion of the coil 4 are fixed to the first wire 2 by fixing materials 11 and 12, respectively. Besides, an intermediate portion (a position near the distal end) of the coil 4 is fixed to the first wire 2 by a fixing material 13. The fixing materials 11, 12, and 13 are each composed of a solder (brazing material). Incidentally, the fixing materials 11, 12, and 13 are not limited to solder, and may each be an adhesive. In addition, the method of fixing the coil 4 is not limited to the use of a fixing material; for example, welding may also be adopted. Besides, for preventing damage to the inside wall of a body lumen such as a blood vessel, the distal end surface of the fixing material 12 is preferably rounded in shape.

In this embodiment, with the coil 4 as above disposed, the first wire 2 is covered with the coil 4, and the contact area between the first wire 2 and a body lumen or the like is small. Therefore, sliding resistance in use can be reduced. Accordingly, the steerability of the guide wire 1 is further enhanced.

Incidentally, in the case of this embodiment, a filamentous material which is circular in cross section is used to constitute the coil 4. However, this configuration is not limitative, and a filamentous material which has, for example, an elliptic or tetragonal (particularly, rectangular) shape or the like shape in section may also be used to constitute the coil 4.

The first wire 2 and the second wire 3 constituting the guide wire body 10 are connected and fixed to each other by welding. This ensures that a high joint strength can be imparted to the joined portion (welded portion) 14 between the first wire 2 and the second wire 3 by a simple method. Therefore, in use of the guide wire 1, a torsional torque and pushing-in force applied from the side of the second wire 3 are securely transmitted to the first wire 2.

The welding method for the first wire 2 and the second wire 3 is not particularly limited; examples of the welding method include friction pressure welding, butt resistance welding such as flash butt welding and upset butt welding, and welding by use of a laser. Among these welding methods, particularly preferred is the butt resistance welding, in view of its merit that a high joint strength can be obtained comparatively easily.

As has been described above, the outside diameter of the proximal end of the first wire 2 is equal to the outside diameter of the distal end of the second wire 3 (the distal end of the tapered portion 16), so that the joined end faces of both wires 2 and 3 accord with each other in shape (preferably, circular shape). Therefore, when both wires 2 and 3 are abutted on each other for welding them, there is generated no step due to a difference in outside diameter.

After both the wires 2 and 3 are joined to each other by welding, a metallic coating layer 6 is formed at the outer peripheral portion of the joined portion 14 of both the wires 2, 3. Now, the shape of the metallic coating layer 6 will be described.

As shown in Fig. 2, the distal side of the joined portion 14 is composed of the first wire 2 which is substantially constant in outside diameter, while the proximal side of the joined portion 14 is composed of the tapered portion 16 of the second wire 3 which is gradually increased in outside diameter in the proximal direction, and the metallic coating layer 6 is so formed as to range across the joined portion 14. The whole part or a part of the metallic coating layer 6 is composed of a tapered section 61 which is gradually increased in outside diameter in the proximal direction.

Here, let the taper angle of the tapered portion 16 (the angle of the outer peripheral surface against the axis of wire) be α and let the taper angle of the tapered section 61 of the metallic coating layer 6 be β, then the relationship of α > β is established (see Fig. 2). In other words, the metallic coating layer 6 is formed in such a manner as to moderate the angular difference between the outer peripheral surface of a proximal end portion of the first wire 2 and the outer peripheral surface of the tapered portion 16 of the second wire 3.

With such a metallic coating layer 6 formed, the sharp change in angle (change in rigidity) across the joined portion 14 (from the distal side to the proximal side or vice versa) can be moderated, and stress concentration on the jointed portion 14 can be excluded or alleviated. Physical properties, particularly, rigidity and elasticity vary smoothly from the second wire 3 to the first wire 2. Therefore, excellent pushability and torque response are displayed across the joined portion 14 of both the wires 2 and 3, and kink resistance thereat is also enhanced.

The metallic coating layer 6 is composed of a solidified matter of a molten metal (melt) generated due to welding between both the wires 2 and 3. Therefore, the material constituting the metal coating layer 6 contains both of the constituent metals of the first wire 2 and the constituent metals of the second wire 3. Particularly, in the case where the first wire 2 and the second wire 3 are formed of the same metallic material or of metallic materials of the same type, as above-mentioned, the material constituting the metallic coating layer 6 also has a metallic composition similar to that or those of the wires 2 and 3.

The metallic coating layer 6 preferably has been obtained by mechanically processing the solidified matter of the molten metal mentioned above. This makes it possible to let the metallic coating layer 6 have a desired shape and desired surface properties, and to further enhance the pushability, torque response, and kink resistance of the guide wire 1. Incidentally, examples of the mechanical processing include grinding, polishing, and laser beam machining, which can be used either singly or in combination of two or more thereof. Besides, a chemical treatment such as etching may be applied in place of the mechanical processing or after the mechanical processing.

The average thickness of the metallic coating layer 6 is not particularly limited. The average thickness is preferably about 1 to 100 µm, more preferably about 1 to 30 µm. If the thickness of the metallic coating layer 6 is too small, the functions of the metallic coating layer 6 mentioned above may fail to be displayed sufficiently. If the thickness is too large, on the other hand, the physical properties (rigidity, elasticity, etc.) may fail to vary smoothly between the first wire 2 and the second wire 3.

The length of the metallic coating layer 6 in the longitudinal direction of wire is not particularly limited, and is preferably about 0.5 to 2.0 mm, more preferably about 0.5 to 1.0 mm.

The metallic coating layer 6 as above is formed, for example, as follows.

The proximal end of the first wire 2 and the distal end of the second wire 3 are put in press contact with each other while impressing a predetermined voltage between the first wire 2 and the second wire 3 by a butt welding machine, for example. By the press contact, a thin layer (e.g., about 0.01 to 50 µm) of melt is formed at the contact portion. When the melt layer is solidified by cooling, the joined portion 14 is formed, whereby the first wire 2 and the second wire 3 are firmly joined to each other. In the instance of this welding, a protuberant portion increased in outside diameter is formed in a predetermined region including the joined portion 14 (e.g., a range of about 0.2 to 8 mm across the joined portion 14). The protuberant portion is formed through solidification of the molten matter of the first wire 2 and the second wire 3.

The protuberant portion of the molten matter is appropriately removed for reshaping (ordering in shape), whereby the metallic coating layer 6 with a desired shape is obtained. Examples of the method of reshaping the protuberant portion include mechanical processings such as grinding, polishing, and laser beam machining, and chemical treatments such as etching. A chemical treatment may be carried out for the purpose of finishing or the like, after the mechanical processing is conducted. By such a reshaping, the outer peripheral surface of the metallic coating layer 6 can be made to be a substantially smooth surface.

While the metallic coating layer 6 has been described as one obtained by reshaping the molten and then solidified matter of the first wire 2 and the second wire 3, the metallic coating layer 6 is not limited to this one; for example, the molten and then solidified matter may be partly left in a projected form or be substantially removed and then another metallic coating may be formed on the surface thereof as above-mentioned. In this case, the another metallic coating can be formed by use of a material of the same type as the materials of the first wire 2 and the second wire 3. In addition, a plastic coating layer (a resin coating layer) may be formed, in place of the metallic coating layer 6. Naturally, a configuration may be adopted in which the whole part or a part of the metallic coating layer 6 is coated with a coating layer of a plastic or the like.

The tapered section 61 of the metallic coating layer 6 is gradually increased rectilinearly in outside diameter in the proximal direction, as shown in Fig. 2. However, the tapered section 61 may have a recessed surface or may have a projected surface.

As shown in Fig. 1, the wire body 10 has resin coating layers 8 and 9 which cover the whole part or a part of the outer peripheral surface (outside surface) of the wire body 10. In the embodiment shown in the figure, the resin coating layers 8 and 9 are provided respectively at the outer peripheries of the first wire 2 and the second wire 3.

These resin coating layers 8 and 9 can be formed for various purposes. One example of the purposes is to reduce the friction (sliding resistance) of the guide wire 1 and to enhance sliding property, thereby enhancing the steerability of the guide wire 1.

Besides, the resin coating layer 8 or 9 may be provided so as to cover the outer periphery of the metal coating layer 6, unlike the case shown in the figure. This makes it possible to further moderate the variation in the outside diameter of the wire body 10 (variation in the taper angle), to further enhance the pushability, torque response, and kink resistance of the guide wire 1, and to enhance the steerability in moving the guide wire 1 in the longitudinal direction.

For reducing the friction (sliding resistance) of the guide wire 1, the resin coating layer 8 and 9 are each preferably formed of a material capable of reducing friction as will be described below. This ensures that the frictional resistance (sliding resistance) between the guide wire 1 and the inside wall of a catheter used with the guide wire 1 is reduced, whereby slidability is enhanced, and the steerability of the guide wire 1 in the catheter is enhanced. In addition, with the sliding resistance of the guide wire 1 lowered, kinking (sharp bending) and torsion of the guide wire 1, particularly, kinking and torsion in the vicinity of the joined portion 14 can be prevented more assuredly from occurring when the guide wire 1 is moved and/or rotated in the catheter.

Examples of the material capable of reducing friction include polyolefins such as polyethylene, polypropylene, etc., polyvinyl chloride, polyesters (PET, PBT, etc.), polyamides, polyimides, polyurethane, polystyrene, polycarbonate, silicone resins, fluororesins (PTFE, ETFE, etc.), and composite materials thereof.

In the case where a fluoro-resin (or a composite material containing the same) as a particular one of the above-mentioned materials is used, it is possible to reduce more effectively the frictional resistance (sliding resistance) between the guide wire 1 and the inside wall of a catheter, to enhance slidability, and to enhance the steerability of the guide wire 1 in the catheter. In addition, by this, kinking (sharp bending) and torsion of the guide wire 1, particularly, kinking and torsion in the vicinity of the welded portion can be prevented more assuredly from occurring when the guide wire 1 is moved and/or rotated in the catheter.

Besides, in the case where a fluoro-resin (or a composite material containing the same) is used, the wire body 10 can be coated with a resin material being in a heated state, by such a method as baking and spraying. This ensures that the adhesion of the resin coating layers 8 and 9 to the wire body 10 will be particularly excellent.

In addition, where the resin coating layers 8 and 9 are each composed of a silicone resin (or a composite material containing the same), the resin coating layers 8 and 9 adhered assuredly and firmly to the wire body 10 can be formed, without heating at the time of forming the resin coating layers 8 and 9 (coating the wire body 10 with the resin). Specifically, where the resin coating layers 8 and 9 are each composed of a silicone resin (or a composite material containing the same), a reaction-curable type material or the like can be used, so that the resin coating layers 8 and 9 can be formed at room temperature. With the resin coating layers 8 and 9 thus formed at room temperature, the coating can be carried out easily, and the guide wire can be operated in the condition where the joint strength between the first wire 2 and the second wire 3 at the jointed portion 14 is maintained sufficiently.

In addition, the resin coating layers 8 and 9 (particularly, the resin coating layer 8 on the distal side) can also be provided for the purpose of enhancing safety at the time of inserting the guide wire 1 into a blood vessel or the like. For this purpose, the resin coating layers 8 and 9 are each preferably formed of a material rich in flexibility (soft material, elastic material).

Examples of the material rich in flexibility include polyolefins such as polyethylene, polypropylene, etc., polyvinyl chloride, polyesters (PET, PBT, etc.), polyamides, polyimides, polyurethane, polystyrene, silicone resins, thermoplastic elastomers such as polyurethane elastomer, polyester elastomers, polyamide elastomers, etc., various rubber materials such as latex rubber, silicone rubber, etc., and composite materials containing two or more of these materials in combination.

Particularly, where the resin coating layers 8 and 9 are each composed of a thermoplastic elastomer or a rubber among the above-mentioned materials, the flexibility of a distal end portion of the guide wire 1 is further enhanced, so that damage to the inside wall of a blood vessel or the like can be prevented more assuredly from occurring at the time of inserting the guide wire 1 into the blood vessel or the like; thus, safety is extremely high.

Each of the resin coating layers 8 and 9 as above may be a laminate body composed of two or more layers. The resin coating layers 8 and the resin coating layer 9 may be composed of the same material or be composed of different materials. For example, a configuration can be adopted in which the resin coating layer 8 located on the distal side of the guide wire 1 is formed of the above-mentioned material rich in flexibility (soft material, elastic material), and the resin coating layer 9 located on the proximal side of the guide wire 1 is formed of the above-mentioned material capable of reducing friction. This configuration makes it possible to achieve both enhancement of slidability (steerability) and enhancement of safety, simultaneously.

The thickness of each of the resin coating layers 8 and 9 is not particularly limited, and is appropriately set in consideration of the purpose(s), the constituent material(s), the forming method(s), and the like of the resin coating layers 8 and 9. Ordinarily, the resin coating layers 8 and 9 each preferably has a thickness (on average) of about 1 to 100 µm, more preferably about 1 to 30 µm. When the thickness of each of the resin coating layers 8 and 9 is too small, the purpose(s) of forming the resin coating layers 8 and 9 may not be fulfilled sufficiently, and exfoliation of the resin coating layers 8, 9 may occur. On the other hand, if the thickness of each of the resin coating layers 8 and 9 is too large, the physical properties of the wire body 10 may be influenced, and exfoliation of the resin coating layers 8, 9 may occur.

Incidentally, in the present invention, the outer peripheral surface (surface) of the wire body 10 may be subjected to a treatment (surface roughening, chemical treatment, heat treatment, or the like) for enhancing the adhesion of the resin coating layers 8 and 9 thereto, or may be provided thereon with an intermediate layer capable of enhancing the adhesion of the resin coating layers 8 and 9.

Preferably, the outside surface of at least a distal end portion of the guide wire 1 is coated with a hydrophilic material. In this embodiment, the outer peripheral surface of the guide wire 1 in the range from the distal end of the guide wire 1 to the vicinity of the proximal end of the tapered portion 16 is coated with a hydrophilic material. This ensures that the hydrophilic material generate lubricity by being wetted, whereby the friction (sliding resistance) on the guide wire 1 is reduced, and slidability is enhanced. Therefore, the steerability of the guide wire 1 is enhanced.

Examples of the hydrophilic material include cellulose polymeric substances, polyethylene oxide polymeric substances, maleic anhydride polymeric substances (for example, maleic anhydride copolymers such as methyl vinyl ether-maleic anhydride copolymer), acrylamide polymeric substances (for example, polyacrylamide, polyglycidyl methacrylate-dimethyl acrylamide (PGMA-DMAA) block copolymer), water-soluble nylon, polyvinyl alcohol, and polyvinyl pyrrolidone.

Such a hydrophilic material, in many cases, displays lubricity due to wetting (water absorption), thereby reducing the frictional resistance (sliding resistance) between the guide wire 1 and the inside wall of a catheter used with the guide wire 1. This enhances the slidability of the guide wire 1, and further enhances the steerability of the guide wire 1 in the catheter.

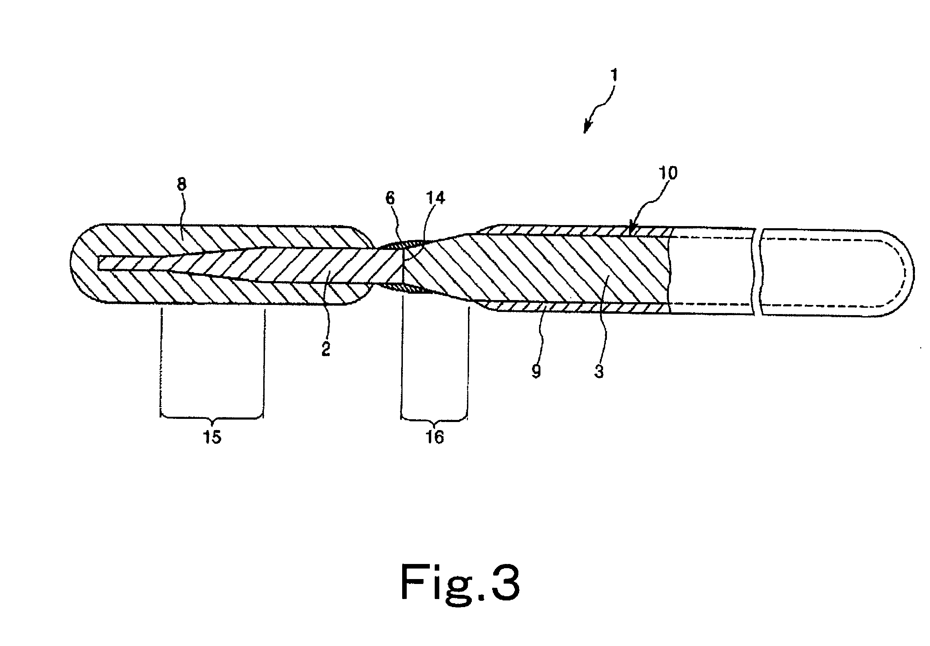

Fig. 3 is a vertical sectional view showing another embodiment of the guide wire according to the present invention. Now, the guide wire shown in Fig. 3 will be described, in which the descriptions of the same items as those of the guide wire shown in Figs. 1 and 2 will be omitted, and description will be centered on the differences between the two guide wires. Besides, for convenience of description, the right side in Fig. 3 is referred to as "the proximal end (proximal side)", and the left side as "the distal end (distal side)". In addition, in Fig. 3, for easier understanding, the guide wire is shown schematically by shortening the guide wire in the longitudinal direction and exaggerating the guide wire in the transverse direction, so that the ratio between the longitudinal size and the transverse size in the figure is different from the real ratio.

The guide wire 1 shown in Fig. 3 is the same as the guide wire 1 shown in Figs. 1 and 2, except for not having the coil 4. The guide wire 1 shown in Fig. 3 has a wire body 10 in which a first wire 2 disposed on the distal side and a second wire 3 disposed on the proximal side of the first wire 2 are joined (connected) to each other by welding, and the outer peripheral portion of the joined portion 14 between the first wire 2 and the second wire 3 is covered with a metallic coating layer 6.

A distal side portion (the outer peripheral surface of the first wire 2) and a proximal side portion (the outer peripheral surface of the second wire 3) of the wire body 10 are covered respectively with resin coating layers 8 and 9.

The purposes, the constituent materials, and the like of the resin coating layers 8 and 9 are the same as above-described. In this embodiment, particularly, the resin coating layer 8 located on the distal side of the guide wire 1 is preferably formed of the above-mentioned material rich in flexibility (soft material, elastic material), while the resin coating layer 9 located on the proximal side of the guide wire 1 is preferably formed of the above-mentioned material capable of reducing friction. This makes it possible to achieve both enhancement of slidability (steerability) and enhancement of safety, simultaneously. In an example of this case, the resin coating layer 8 is formed of polyurethane, and the resin coating layer 9 is formed of a fluoro-resin (PTFE, ETFE, or the like).

As shown in Fig. 3, a proximal end portion of the resin coating layer 8 is terminated on the distal side relative to the proximal end of the first wire 2. A distal end portion of the resin coating layer 9 is terminated on the proximal side relative to the distal end of the second wire 3. The proximal end portion of the first wire 2 and the distal end portion of the second wire 3 are exposed, with the joined portion 14 located therebetween. In the case of a guide wire used with an endoscope, the proximal end portion of the resin coating layer 8 and the first wire 2 are different in color, so that the motions of the guide wire can be recognized.

The outside diameter of the resin coating layer 8 is equal to the outside diameter of the resin coating layer 9.

The resin coating layer 8 covers the distal end of the first wire 2 so as not to expose the distal end; moreover, the distal end of the resin coating layer 8 is preferably rounded in shape. This makes it possible to prevent more effectively the damages to the inside wall of a body lumen such as a blood vessel at the time of inserting the guide wire 1 into the body lumen, and thereby to enhance safety.

In the resin coating layer 8, a filler (particles) of a contrast material (the above-mentioned radiopaque material) may be mixed, so as to constitute a radioscopically effective (contrast) portion.

The outside surface of at least a distal end portion of the guide wire 1 is preferably coated with a hydrophilic material. In this embodiment, the outer peripheral surface of the guide wire 1 in the range from the distal end of the guide wire 1 to the vicinity of the proximal end of the tapered portion 16 is coated with a hydrophilic material. This ensures that the hydrophilic material generates lubricity by being wetted, whereby the friction (sliding resistance) on the guide wire 1 is reduced, and slidability is enhanced.
Therefore, the steerability of the guide wire 1 is enhanced.

The coating of the hydrophilic material may be formed only on a part of the outside surface of the resin coating layer 8 or the whole area of the outside surface. Incidentally, specific examples of the hydrophilic material are the same as above-mentioned.

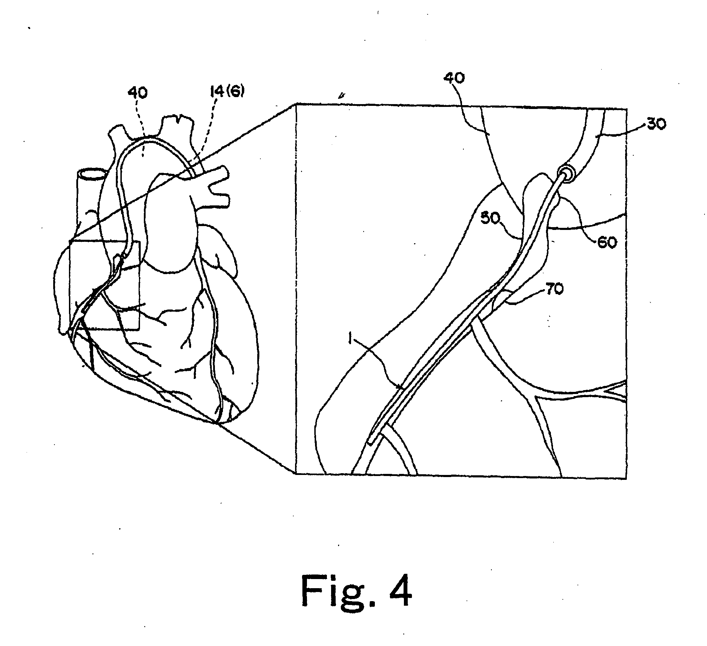

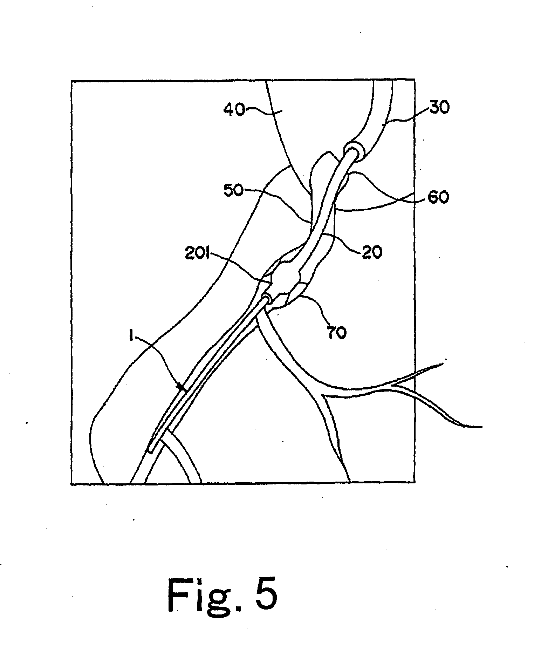

Figs. 4 and 5 are diagrams showing the use conditions in the case where the guide wire 1 according to the present invention is used for PTCA.

In Figs. 4 and 5, symbol 40 denotes an aortic arch, symbol 50 denotes a right coronary artery of a heart, symbol 60 denotes a right coronary artery orifice, and symbol 70 denotes an angiostenosis portion (lesion portion). In addition, symbol 30 denotes a guiding catheter for securely guiding the guide wire 1 to the right coronary artery through a femoral artery, and symbol 20 denotes a stenosis portion dilating balloon catheter having a dilatable and contractable balloon 201 at its distal end portion. The following operations are conducted under radioscopic observation.

As shown in Fig. 4, the distal end of the guide wire 1 is protruded from the distal end of the guiding catheter 30, and is inserted through the right coronary artery orifice 60 into the right coronary artery 50. Further, the guide wire 1 is moved forward to be inserted into the right coronary artery 50, starting from its distal end, and is stopped when the distal end has come beyond the angiostenosis portion 70. By this, the path for the balloon catheter 20 is secured. Incidentally, in this instance, the joined portion 14 (the metallic coating layer 6) of the guide wire 1 is located on the descending aorta side of the aortic arch 40 (in the living body).

Next, as shown in Fig. 5, the distal end of the balloon catheter 20 inserted from the proximal end side of the guide wire 1 is protruded from the distal end of the guiding catheter 30, is further moved forward along the guide wire 1, to be inserted through the right coronary artery orifice 60 into the right coronary artery 50, and is stopped when the balloon 201 has reached the position of the angiostenosis portion 70.

Subsequently, a balloon dilating fluid is fed in from the proximal end side of the balloon catheter 20, to dilate the balloon 201, thereby dilating the angiostenosis portion 70. In this manner, the deposits such as cholesterol deposited on the blood vessel at the angiostenosis portion 70 is physically pushed wide open, whereby bloodstream inhibition is dissolved.

While the guide wire according to the present invention has been described above based on the embodiments shown in the drawings, the invention is not limited to the embodiments. Each part constituting the guide wire can be replaced by a part which has an arbitrary configuration and which can display the same function as that of the replaced part. Besides, arbitrary components may be added.

In addition, the use of the guide wire according to the present invention is not limited to the use in PTCA described above. For example, the guide wire can be used in angiography, endoscopic procedure, and the like.

## Claims

1. A guide wire comprising a wire body (10) including a first wire (2) disposed on the distal side, and a second wire (3) disposed on the proximal side of said first wire (2), the proximal end of said first wire (2) and the distal end of said second wire (3) being joined to each other by welding, wherein
a proximal end portion of said first wire (2) is substantially constant in outside diameter along the longitudinal direction, whereas a distal end portion of said second wire (3) is a tapered portion (16) gradually increased in outside diameter in the proximal direction from the distal end thereof, and
the proximal end of said first wire (2) and the distal end of said second wire (3) are equal in outside diameter,
**characterized in that**
a coating layer (6) is provided on an outer peripheral portion of a joint surface (14) between said first wire (2) and said second wire (3), wherein said coating layer (6) has a tapered section (61) gradually increased in outside diameter in the proximal direction, and the taper angle of this tapered section (61) of said coating layer (6) is smaller than the taper angle of said tapered portion (16) of said second wire (3) to moderate the angular difference between the outer peripheral surface of said proximal end portion of said first wire (2) and the outer peripheral surface of said tapered portion (16).

2. The guide wire as set forth in claim 1, wherein said first wire (2) and said second wire (3) are formed of the same metallic material or formed of metallic materials of the same type.

3. The guide wire as set forth in claim 2, wherein said first wire (2) and said second wire (3) are each formed of a Ni-Ti base alloy.

4. The guide wire as set forth in any one of the preceding claims, wherein said coating layer (6) is a metallic coating layer (6).

5. The guide wire as set forth in claim 4, wherein said metallic coating layer (6) is formed from a molten metal generated upon the welding of said first wire (2) and said second wire (3).

6. The guide wire as set forth in claim 5, wherein said metallic coating layer (6) has been reshaped by mechanically processing a solidified matter of said molten metal.

7. The guide wire as set forth in any one of claims 4 to 6, wherein said metallic coating layer (6) has an average thickness of 1 to 100 µm.

## Patentansprüche

1. Führungsdraht mit einem Drahtkörper (10) mit einem ersten Draht (2), der an der körperfernen Seite angeordnet ist, und einem zweiten Draht (3), der an der körpernahen Seite des ersten Drahts (2) angeordnet ist, wobei das körpernahe Ende des ersten Drahts (2) und das körperferne Ende des zweiten Drahts (3) miteinander durch Schweißen verbunden sind, wobei
ein körpernaher Endabschnitt des ersten Drahts (2) entlang der Längsrichtung im Wesentlichen einen konstanten Außendurchmesser hat, wohingegen ein körperferner Endabschnitt des zweiten Drahts (3) ein verjüngter Abschnitt (16) ist, dessen Außendurchmesser in der körpernahen Richtung von seinem distalen Ende her allmählich vergrößert ist, und
das körpernahe Ende des ersten Drahts (2) und das körperferne Ende des zweiten Drahts (3) einen gleichen Außendurchmesser aufweisen,
**dadurch gekennzeichnet, dass**
eine Beschichtungslage (6) an einem Außenumfangsabschnitt einer Verbindungsfläche (14) zwischen dem ersten Draht (2) und dem zweiten Draht (3) vorgesehen ist, wobei die Beschichtungslage (6) einen verjüngten Abschnitt (61) hat, dessen Außendurchmesser in der körpernahen Richtung allmählich vergrößert ist, und wobei der Verjüngungswinkel dieses verjüngten Abschnitts (61) der Beschichtungslage (6) kleiner als der Verjüngungswinkel des verjüngten Abschnitts (16) des zweiten Drahts (3) ist, um die Winkeldifferenz zwischen der Außenumfangsfläche des körpernahen Endabschnitts des ersten Drahts (2) und der Außenumfangsfläche des verjüngten Abschnitts (16) im Maß zu halten.

2. Führungsdraht gemäß Anspruch 1, wobei der erste Draht (2) und der zweite Draht (3) aus demselben metallischen Material gemacht sind oder aus metallischen Materialien derselben Art gemacht sind.

3. Führungsdraht gemäß Anspruch 2, wobei der erste Draht (2) und der zweite Draht (3) jeweils aus einer Ni-Ti Basislegierung gemacht sind.

4. Führungsdraht gemäß einem der vorangehenden Ansprüche, wobei die Beschichtungslage (6) eine metallische Beschichtungslage (6) ist.

5. Führungsdraht gemäß Anspruch 4, wobei die metallische Beschichtungslage (6) aus einem geschmolzenen Metall gemacht ist, welches in Folge des Schweißens des ersten Drahts (2) und des zweiten Drahts (3) erzeugt wird.

6. Führungsdraht gemäß Anspruch 5, wobei die metallische Beschichtungslage (6) durch mechanisches Bearbeiten eines verfestigten Materials des geschmolzenen Metalls neu gestaltet wurde.

7. Führungsdraht gemäß einem der Ansprüche 4 bis 6, wobei die metallische Beschichtungslage (6) eine mittlere Dicke von 1 bis 100 µm aufweist.

## Revendications

1. Fil de guidage comprenant un corps (10) de fil comportant un premier fil (2) disposé sur le côté distal, et un deuxième fil (3) disposé sur le côté proximal dudit premier fil (2), l'extrémité proximale dudit premier fil (2) et l'extrémité distale dudit deuxième fil (3) étant reliées par soudure, où
une partie d'extrémité proximale dudit premier fil (2) a un diamètre externe essentiellement constant le long de la direction longitudinale, tandis qu'une partie d'extrémité distale dudit deuxième fil (3) est une partie conique (16) ayant un diamètre externe qui augmente graduellement dans la direction proximale depuis son extrémité distale, et
le diamètre externe de l'extrémité proximale dudit premier fil (2) est égal au diamètre externe de l'extrémité distale dudit deuxième fil (3),
**caractérisé en ce que**
une couche de gainage (6) est prévue sur une partie périphérique externe d'une surface de raccordement (14) entre ledit premier fil (2) et ledit deuxième fil (3), où ladite couche de gainage (6) a une section conique (61) ayant un diamètre externe qui augmente graduellement dans la direction proximale, et l'angle conique de cette section conique (61) de ladite couche de gainage (6) est inférieur à l'angle conique de ladite partie conique (16) dudit deuxième fil (3) afin de modérer la différence angulaire entre la surface périphérique externe de ladite partie d'extrémité proximale dudit premier fil (2) et la surface périphérique externe de ladite partie conique (16).

2. Fil de guidage selon la revendication 1, dans lequel ledit premier fil (2) et ledit deuxième fil (3) sont formés du même matériau métallique ou sont formés de matériaux métalliques du même type.

3. Fil de guidage selon la revendication 2, dans lequel ledit premier fil (2) et ledit deuxième fil (3) sont chacun formé d'un alliage à base de Ni-Ti.

4. Fil de guidage selon l'une quelconque des revendications précédentes, dans lequel ladite couche de gainage (6) est une couche de gainage métallique (6).

5. Fil de guidage selon la revendication 4, dans lequel ladite couche de gainage métallique (6) est formée à partir d'un métal en fusion généré lors du soudage dudit premier fil (2) et dudit deuxième fil (3).

6. Fil de guidage selon la revendication 5, dans lequel ladite couche de gainage métallique (6) a été remodelée en traitement de manière mécanique des matières solidifiées dudit métal en fusion.

7. Fil de guidage selon l'une quelconque des revendications 4 à 6, dans lequel la couche de gainage métallique (6) a une épaisseur moyenne de 1 à 100µm.
